# EUROPEAN PATENT APPLICATION

(11) **EP 0 896 965 A1**
(43) Date of publication of application: **17.02.1999**
(21) Application number: 97113995.1
(22) Date of filing: 13.08.1997
(51) Int. Cl.: C07K 7/06, C07K 14/47, A61K 38/08, A61K 38/18

(54) **Inhibitors of G-protein-beta, gamma subumit function**

(71) Applicant: BOEHRINGER MANNHEIM GMBH, 68298 Mannheim (DE)
(72) Inventor: The designation of the inventor has not yet been filed

(57) **Abstract**

G-protein βγ-subunits (Gβγ) act as active transmembrane signaling components. It is shown here that the high affinity and inhibitory action of phosducin for Gβγ is largely due to a small region (amino acids 217-231) in its C-terminus. Corresponding peptides including this region disrupt Gβγ-Gα-interactions, but also other Gβγ-mediated functions. This region represents a model switch that can turn off Gβγ. The knowledge of the region in phosducin and the part of Gβγ interacting with this region of phosducin enables the design of small compounds which act as Gβγ antagonists.

## Description

The invention refers to inhibitors and activators of G-proteins. The βγ-complexes are dimers comprising one β subunit and one γ subunit, each of which occurs in more than one isoform. The dimers may form from every possible combination of one β subunit and one γ subunit. The compounds of the invention are capable of inhibiting or damping the function of the βγ-complex of G-proteins. The compounds of the invention are of peptidic or nonpeptidic nature, and will attenuate signals of G-protein-coupled receptors in all or specific organs and cells. This effect is useful in diseases where
a) signal transduction via such receptor/G-protein interaction is pathologically increased, for example in many genetic hormonal diseases caused by activating mutations in corresponding receptors, or in tumors caused by similar mutations,
b) receptors are pathologically activated due to an increase in their respective endogenous ligands, for example in hormonal, cardiovascular, nervous system or other diseases involving increased secretion of hormones or neurotransmitters,
c) receptor/G-protein binding in the patient is normal, but a decrease in their activity would be beneficial to the patient. This is analogous to blockage of β-adrenergic receptors in the treatment of arterial hypertension or the prophylaxis of myocardial infarction, or to counteract imbalances of other receptor systems, such as signaling disorders in the central nervous system including psychiatric or degenerative diseases,
d) the effect of exogenous receptor agonists and other activators of the corresponding signaling pathways (such as drugs or poisons) are to be counteracted by inhibiting G-protein function.

Examples for the above mentioned disease are, inter alia, high blood pressure, coronal diseases affected by α- or β-receptor antagonists, allergies, ulcer related diseases affected by histamine receptor antagonists, schizophrenia (e.g. D2-dopamine-receptor antagonist affected) and other diseases of the central nervous system (M. Alzheimer and psychoses).

A further embodiment of the invention is to provide the tools of molecular modeling of Gβγ-complex inhibitors and attenuators, by disclosing the relevant sites both of βγ-complex and of the relevant amino acids in phosducin. With this knowledge a person skilled in the art can design peptides, peptidomimetics and chemical entities which fit at the βγ-complex site to affect the biological properties of said complex. Of special interest for the development of pharmaceutical compounds are such compounds with a molecular weight which is less than phosducin(204-246). Preferred are compounds with a molecular weight which is identical or less than phosducin(204-231) or phosducin(217-246), more preferred with a molecular weight identical or less than phosducin(213-233).

G-proteins act as signal transducers of many membrane-bound, heptahelical receptors. The G proteins consist of a GTP-binding α subunit and a tightly bound complex composed of β and γ subunits. When G proteins are activated by their respective receptors, the α subunits exchange GDP for GTP, and this causes dissociation of αGTP and the βγ complex, both of which can then regulate the activity of various effector molecules such as adenylyl cyclases or ion channels (reviewed in Birnbauer 1992, Hepler and Gilman 1992, Neer 1995, Mueller and Lohse 1995). G-protein-mediated signaling is subject to a variety of regulatory controls. Most of these are exerted at the receptor level (reviewed by Hausdorff et al. 1990, Lohse 1993), but more recently evidence has been accumulating that regulation can also occur at the G-protein level.

Several proteins have been described to interact with G-proteins and to alter their activity. The growth cone protein GAP-43 and the RGS (regulators of G-protein signaling) family members have been shown to activate the a subunits of Gₒ, or the Gᵢ family (Strittmatter et al. 1991; Berman et al, 1996, Hunt et al. 1996, Watson et al. 1996). Gₒ, Gᵢ, Gₛ are G-proteins having an α-subunit of the s (stimulates adenylyl cyclase), i (inhibits adenylyl cyclase) or o (other, presumably couples to ion channels) type (Offermanns and Schulk 1994). A number of other proteins, in particular the β-adrenergic receptor kinases (Pitcher et al. 1992, Koch et al. 1993, Mueller et al, 1993), have been shown to bind to G-protein βγ-subunits and this binding has been suggested to be mediated by the C-terminal helix plus adjacent sequences of a conserved structural motif called pleckstrin homology (PH) domain (Touhara et al. 1994). A consensus motif Gln/Asn-X-X-Glu/Asp-Arg/Lys has been identified in several βγ-binding effector proteins (β-adrenergic receptor kinase, adenylyl cyclase type II, phospholipase β-3 and atrial K⁺-channels) and is considered to be responsible for mediating this interaction (Chen et al. 1995).

Phosducin is a cytosolic protein which also belongs to the group of Gβγ-binding proteins and which appears to act as a regulator of signaling via G-proteins (Bauer et al. 1992. Lee et al. 1992). It occurs in very high concentrations in the retina (Lee et al. 1987) and the developmentally related pineal gland (Reig et al. 1990), but is also expressed in brain and many other tissues at a concentration of about 1 µM (Bauer et al. 1992, Danner and Lohse 1996). These concentrations are sufficient to inhibit G-protein-mediated activation of effectors, such as the receptor-induced stimulation of adenylyl cyclase via Gₛ, (Denner and Lohse 1996, Schulz et al. 1996). Upon phosphorylation of phosducin by protein kinase A, the inhibitory effects of phosducin on G proteins are essentially lost (Bauer et al, 1992, Yoshida et al. 1994, Hawes et al. 1995).

Phosducin has a high affinity for Gβγ, which is in the range of 20 nM (Bauer et al. 1992, Mueller et al. 1996). Two independent studies have suggested that the N-terminal 63 (Xu et al. 1995) or 105 (Hanges et al. 1994) amino acids mediate this interaction. However, the short variant of a phosducin like protein (PhLP), which lacks the N-terminus but is otherwise about 50% identical to phosducin, also interacts with Gβγ with an affinity only 5-fold lower than that of phosducin (Schroeder and Lohse 1996). This suggests the presence of a Gβγbinding region in the large overlapping domain of these two proteins. The present invention discloses the identification and the structure of a small critical region in the C-terminus of phosducin that mediates Gβγ binding.

G-protein βγ-subunits (Gβγ) act as active transmembrane signaling components. Their function has recently been observed to be regulated by the cytosolic protein phosducin. It is shown here that the high affinity and inhibitory action of phosducin for Gβγ is largely due to a small region (amino acids 217-231) in its C-terminus. Corresponding peptides including this region disrupt Gβγ-Gα-interactions, and also other Gβγ-mediated functions. From the NMR structure it has been determined that the segment 217 to 226 of phosducin is particularly important. The sequence FLNEYGLL is the active core of phosducin. The NMR structure of a peptide encompassing this region shows a loop exposing the side chains of Glu223 and Tyr224, and peptides with a substitution of either of these amino acids show a complete loss of activity towards Gₒ. These above mentioned regions represents a model switch that can turn off Gβγ. The knowledge of the region in phosducin and the part of Gβγ interacting with this region of phosducin enables the design of inhibitors including small compounds which act as Gβγ antagonists to prevent G-protein related diseases. The Purpose of the invention is to provide molecules which interact with Gβγ in an equivalent manner as E223 and Y224 of phosducin do. An equivalent manner means that they interact at the same region of the Gβγ-complex. The mode of interaction is preferably allosteric. Same region means within the resolution of the crystalline structure, i.e. within 5 A, more preferred within 2.4 A and most preferred within 1A.

### Brief Description of the Drawings

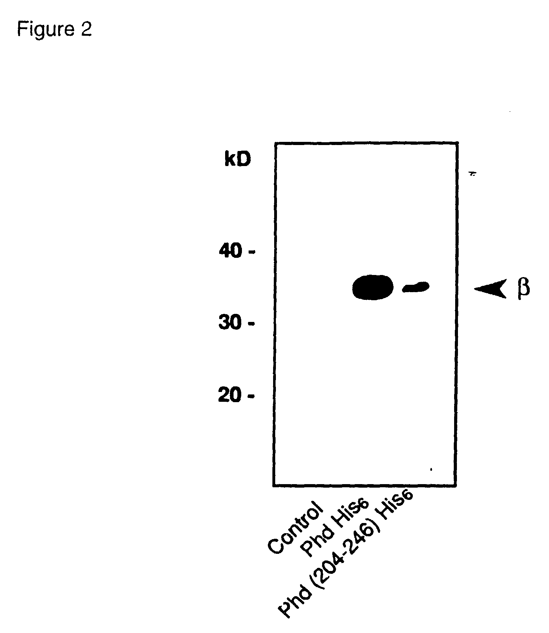

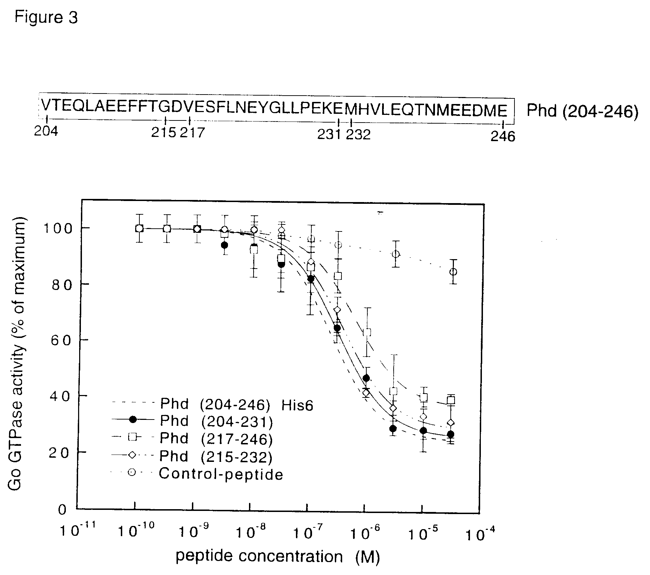

Figure 1
   Inhibition of GTPase activity of Go by His6-tagged phosducin and its N-terminally truncated variants. The top bar shows the identities (black) and similarities (gray) and gaps (brackets) between phosducin and the long form of phosducin-like protein (PhLP - taken from Schroeder and Lohse 1996). The size of the short form of PhLP is indicated by the line on the top. Analysis with nonlinear curve fitting gave the following IC50-values: phosducin 15±1 nM, phosducin(64-246) 33±3 nM, phosducin( 138-246) 25±2 nM, phosducin(172-246) 95±13 nM, phosducin(204-246) 240±20 nM. Data are mean ± SEM of 3 independent experiments
Figure 2
   Direct binding of His6-tagged phosducin and phosducin(204-246) to Gβγ. Purified Gβγ (130 pmol) was incubated alone (left lane) or with 250 pmol phosducin (middle lane) or phosducin(204-246) on ice, and the His6-tagged proteins were then bound to Ni-NTA beads and pelleted. After washing, the Gβγ retained in the pellet was detected by SDS-polyacrylamide gel electrophoresis and Western blotting with antibodies directed against Gβ.
Figure 3
   Inhibition of G₀GTPase activity by synthetic peptides (top) derived from the C-terminus of phosducin. The basal GTPase activity of Gₒ was assayed in solution (in the presence of 0.1 % Lubro and 25 mM MgSO₄. The dotted line represents the effects of the His6-tagged phosducin(204-246). An unrelated peptide (taken from the first intracellular loop of the human β2-adrenergic receptor KTAIAKFERLQTVTNYFITSK) served as a control. IC₅₀-values were: phosducin(204-231) 320±45 nM, phosducin(217-246) 630±140 nM, phosducin(215-232) 380±30 nM. Data are mean ± SEM of 3 independent experiments.
Figure 4
   Inhibition of Gβγ-mediated effects by phosducin(215-232).
   a) Inhibition of Gβγ-stimulated adenylyl cyclase-2 activity in the presence of 100 nM G_{sα} and 100 nM Gβγ. 5 µg (protein) of membranes prepared from Sf9 cells infected with baculovirus for adenylyl cyclase-2 were incubated for 10 min at 30 C with 100 nM GTPγS-activated purified Gsα (α-unit of Gs) and then for 20 min on ice with 100 nM Gβγ, followed by determination of adenylyl cyclase activity.
   b) Inhibition of rhodopsin phosphorylation by the β-adrenergic receptor kinase in the presence Gβγ. The activity of βARK was determined using rhodopsin in rod outer segments the substrate as in Schroeder and Lohse 1996. The protein concentrations were 1 µM rhodopsin, 2.5 nM βARK-1 and 20 nM Gβγ, and incubations, lasted for 6 min at 30 C. The concentration of phosducin(215-232) was 100 µM..
   c) Inhibition of Gβγ(600 nM)-stimulated phospholipase C-β2 activity. Phospholipase C-β2 activity was determined with a truncated version of PLC-β2 (PLC-mut) as described by Dietrich et al. (1994). [³H]Phosphatidyl 4,5-bisphosphate served as substrate. When present, the concentration of Gβγ was 600 nM and that of Phd(215-232) 100 µM. Activity is expressed as pmoles of inositol 1.4.5-triphosphate formed per minute.
Figure 5
   Amide region of a 500 MHz WATERGATED NOESY spectrum of the peptide phosducin(204-231) in 10 mM Phosphate buffer containing 100 mM NaCI and 10% D₂O at pH = 6.5 and 277 K. The mixing time used was 250 ms. Numbers denote the corresponding amino acid residues. The strong NH-NH NOEs indicate an ordered state in the middle part of the peptide phosducin(204-231) and were also observed using a mixing time of 40 ms.
Figure 6
   Peptide sequence of phosducin(204-231) and summary of short and medium range NOEs observed in NOESY spectra. Line thickness is proportional to the signal intensity. NOEs between side chains are not shown. Arrows are used to describe the secondary structure elements.
Figure 7
   Superposition of 20 from 80 calculated NMR structures with the lowest energy comprising residues phosducin(215-226). Only the protein backbone, hydrophobic residues (V217, F220, L221, L226, L227) and the exposed residues Glu 223 (E223) and Tyr 224 are shown. These 20 structures fit with an average backbone: 〈R.m.s.d〉 of 0.69 A. The critical contacts between phosducin and β-transducin are phosducin E223 / β-transducin T47, phosducin E223 / β-transducin R46, phosducin Y224 / β-transducin G310, phosducin Y224 / β-transducin R46.
Figure 8
   Inhibition of G₀GTPase activity by phosducin(215-231) and variants. The effects of the peptides were assayed as in Figure 2 at a concentration of 30 µM. The inhibition by the same concentration of the unrelated peptide KTAIAKFERLQTVTNYFITSK (see Fig. 3) was subtracted as non-specific background. WT: wild-type sequence (see Fig. 3); FLY->A:F220, L221, Y224, L226, L227 all replaced by Ala. Data are mean and SEM of 3 independent experiments.

The structural and functional basis for the interaction of various proteins with Gβγ has recently attracted much interest, and conflicting models postulating either a common mode of all Gβγ-binding proteins or separate and distinct ways of interaction have been put forward. Models assuming a common mode would be strengthened by the existence of defined Gβγ-binding consensus motifs. The data presented here indicate the C-terminus of phosducin contains a minimal Gβγ binding region which conveys submicromolar affinity for Gβγ and disrupts several Gβγ-mediated functions. Other studies using Glutathione-S-Transferase(GST)-phosducin fusion proteins (Hawes et al. 1995, Xu et al. 1995) found no major contribution of regions other than the N-terminus to Gβγ-binding. However, experiments comparing phosducin with PhLP (Schroeder and Lohse 1996) as well as the present truncation studies revealed only a 2- to 5-fold increase in the affinity for Gβγ caused by the presence of phosducin's N-terminus, and clearly indicate that a major site of interaction lies in the C-terminal region. The most likely explanation for this discrepancy is that the presence of an N-terminal GST-moiety disturbed the Gβγ-binding function of the C-terminal region.

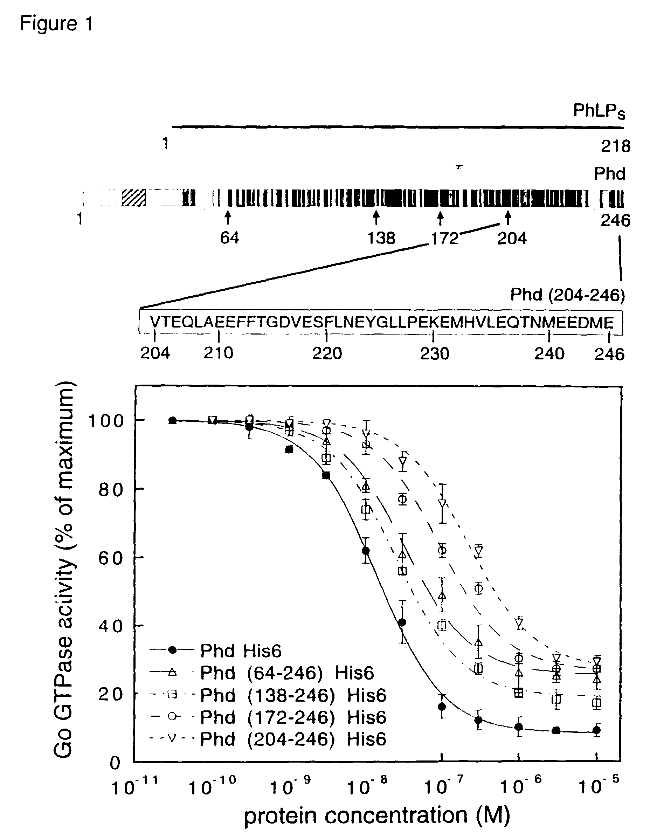

A consensus Motif N/Q-X-X-D/E-R/K has been proposed for Gβγ-binding based on Gβγ-interactions with state of the art inhibitors (Chen et al. 1995). Even though the sequence of phosducin(217-231) contains these amino acids, it lacks the correct spacing to correspond to this consensus motif. This is supported by the fact that the critical sequence FLNEYGLL of phosducin has no identical or similar counterpart in any eukaryotic protein contained in GenBank or SwissProt data banks (data not shown). This suggests that the proposed consensus motif for Gβγ-binding is not utilized by phosducin, even though the affinity of the phosducin region has a much higher affinity for Gβγ than peptides containing this proposed consensus motif The recently completed X-ray structure of phosducin together with the βγ-subunits of transducin indicates the presence of large interfaces between these proteins (Gaudet et al. 1996). This structure shows interactions of Gβγ with the N-terminus as well as the C-terminal domain of phosducin which are compatible with the progressive loss of affinity in the truncation studies (Figure 1). However, within these large interfaces, the short interacting region identified by the present invention provides the essential contribution to Gβγ-binding. The fact that this region is of similar character in the X-ray structure and in the peptide together with the high affinity of the peptide for Gₒ support the biological importance of this structure. Interestingly, this region of phosducin binds to the side of blades 1 and 7 of the β-propeller, and thus a region distinct from the interface with Gα. This precludes a direct competition between the small phosducin peptides and Gα. However, three loops contained in blades 1 and 3 of Gβ are altered in the phosducin-Gβγ complex (Gaudet et al. 1996). This raises the possibility that the C-terminus of phosducin induces a conformational change in Gα which inhibits coupling between Gα and Gβγ.

The results of figures 1 and 3 show that the Gα-Gβγ interaction was more sensitive to the effects of the phosducin peptides than Gβγ-effector interactions (Figure 4). This is contrary to the concept of a common Gβγ binding motif (Chen et al. 1995), Gα and effector molecules have different binding modes to Gβγ. The apparent allosteric regulation of the Gα-Gβγ interaction by phosducin represents a third and novel type of interaction with Gβγ. Rather than competing for a common binding surface on Gβγ (Weng et al. 1996), this critical sequence contained in phosducin seems to act as a small switch converting Gβ into an "off" state in which it does not interact with Gα (and, at higher concentrations, with effectors). The small size of this region together with its defined structural elements constitute a basis for the development of Gβγ-antagonists.

### Example 1

### Mapping of the region in phosducin mediating inhibition of G₀GTPase

Various N-terminally truncated variants of phosducin were generated to map its Gβγ-binding site. The first deletion (amino acids 1-63) encompasses the N-terminal Gβγ-binding site according to Xu et al. (1992), and the resultant protein contains the regions of phosducin similar to the short variant of PhLP (Figure 1). Further deletions were done to cover the remaining structure. The full length and truncated proteins, expressed and purified as C-terminally His6-tagged proteins were all capable of inhibiting the GTPase activity of Gₒ (Figure 1). These inhibitory effects were the same for the mastoparan analogue (Mas-7) stimulated (Higashijima et al. 1990) activity of Gₒ reconstituted into phospholipid vesicles (figure 1) and the basal activity of Gₒ in detergent containing buffer (not shown). Phosducin itself inhibited the GTPase activity of Gₒ by ∼ 90% with an IC₅₀ -value of 15 nM. Deletion of the first 63 amino acids causes only a 2-fold loss in affinity, which contrasts with the results from transfection assays obtained by Xu et al. (1992). Further N-terminal deletions caused further modest reductions in the potency and efficacy, but even the very C-terminus of phosducin (aa. 204-246) had an IC₅₀-value of about 250 nM indicating an affinity comparable to that of many intact Gβγ-binding proteins.

Phosducin itself as well as its small C-terminus displayed direct Gβγ-binding: Co-incubation of purified Gβγ with His-tagged phosducin in solution allowed specific co-precipitation of Gβγ with a His6-binding resin (Figure 2). When His-tagged phosducin(204-246) was used instead of phosducin, such a signal was also obtained, even though the intensity of the signal was reduced in accordance with the lower affinity of the short fragment.
Further mapping of the Gβγ-binding site was done with synthetic peptides derived from the C-terminus of phosducin (Figure 3). Two overlapping peptides comprising the active C-terminus as defined above both inhibited the GTPase activity of Gₒ. The phosducin(204231) peptide had a slightly higher efficacy and potency than the peptide representing the very C-terminus (aa. 217-246). These data suggested that the overlapping region of the two peptides contains the essential Gβγ-binding site. This conclusion was confirmed by the fact that a peptide containing this overlapping region (215-232) was as potent as the larger peptides in inhibiting GoGTPase, with an IC₅₀-value of about 380 nM. Considering these peptide data together, aa. 217-231 contain the essential Gβγ-binding region.

### Construction and purification of phosducin-His6 fusion proteins

In order to generate phosducin with a C-terminal His6-tag, a synthetic cDNA fragment corresponding to a 41 bp *Nsil-BamHI* fragment at the very C-terminus of phosducin and encoding for six additional consecutive histidine residues in front of the stop codon of phosducin was ligated into the corresponding position in the bacterial expression vector pET-Phd (Bauer et al. 1992) containing the coding region of bovine phosducin. N-terminal deletions of phosducin-His6 were generated via polymerase chain reactions using 5'-primers encoding the new N-termini and 3'-primers corresponding to the region next to the stop codon. All cDNA constructs were verified by sequencing. The vectors were introduced into the *E.coli* strain BL21 (DE3)pLysS and induced as described (Bauer et al. 1992). The induced cells were lysed in 50 mM Na-phosphate buffer pH 7.4 by sonication. The lysate was centrifuged at 19,000 g for 30 minutes and the His6-tagged proteins were purified from the supernatant to >95% homogeneity by chromatography on Ni-NTA columns (Quiagen).

### Peptide synthesis

Peptides were synthesized automatically on a MilliGen 9050 peptide synthesizer by the solid phase method using standard Fmoc chemistry in the continuous flow mode. Final cleavage from the resin and deprotection of side chains was achieved by a mixture of 88% trifluoroacetic acid / 5% phenol / 5% Water / 2% trlisopropyisilane for 3 h, and purification was done by preparative HPLC giving products of>95% purity by HPLC. Peptides were characterized by matrix-assisted laser desorption/ionization mass spectrometry which gave the expected [M+H] peaks, and amino acid analysis.

### Determination of the GTPase activity of Go

The steady state GTPase activity of G₀ (purified from bovine brain according to Sternweis and Robishaw 1984) was measured by determining the release of [³²P] from [γ-³²P]GTP as described earlier (Lohse et al. 1992). Unless indicated otherwise. 0.2 pmol of G₀ was assayed in the presence of 25 mM MgSO₄ in a reaction volume of 100 µl. Phosducin-His6 proteins or phosducin peptides were present at the indicated concentrations and the concentration-response curves were analyzed with the Hill equation as described (Lohse et al. 1986). The incubations lasted for 30 min at 30 C and were terminated by addition of 500 µl of 1 % charcoal in 2 mM NaH₂PO₄, pH 2. The MAS-7-stimulated (Higashijima et al. 1990) GTPase activity of Go was measured with Go (0.1 pmol per tube) reconstituted into crude phosphatidylcholine vesicles as described (Lohse et al. 1992). GTPase assays were performed as described above in the presence of 50 pM MAS-7.

### Detection of Gβγ,-binding to His6-tagged phosducins

To determine their Gβγ-binding capability, purified phosducin-His6 proteins (250 pmol) were incubated with 130 pmol Gβγ purified from bovine brain in phosphate-buffered saline containing 0.05% cholate (140 mM NaCI, 30 mM KCI, 6.5 mM Na₂HPO₄, pH 7.3) for 20 min on ice (Pitcher et al. 1992). The fusion proteins were then bound to 30 µl of Ni-NTA resin (Qiagen). The beads were washed in the same buffer with intervening short centrifugations, and the bound Gβγ was detected by taking up the beads in SDS-sample buffer followed by SDS-polyacrylamide gel electrophoresis and Western blotting with antibodies against the ∼ subunit (Signal Transduction Laboratories). Peroxidase-coupled secondary antibodies and enhanced chemiluminescence reagents (Amersham) were used to develop the blots.

### Example 2

### Inhibition of other Gβγ-mediated effects

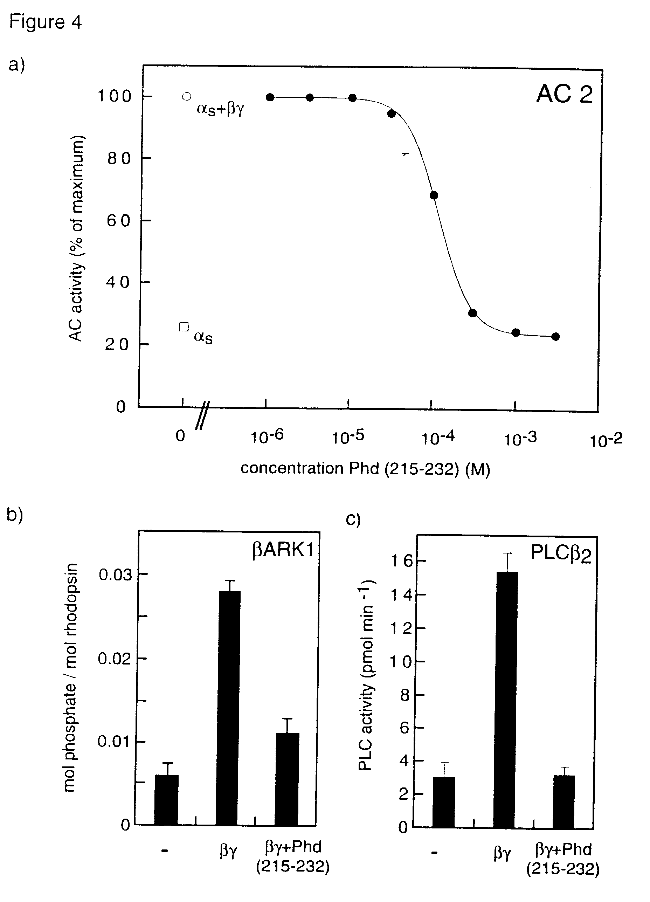

Gβγ interact with multiple proteins other than Gα, and therefore the peptide phosducin(215-232) was tested for its ability to antagonize Gβγ-effects on various effector proteins. Since these interactions require cell membranes, it was determined that the IC50-value of this peptide on GoGTPase in brain membranes was about 20 µM (data not shown), indicating that membranes considerably decrease the peptide's potency. Gβγ-stimulated activity of adenylyl cyclase in the membranes of Sf9 cells was inhibited by phosducin (215-232) with an IC50-value of 100 µM (figure 4a). In contrast, the activity stimulated with 100 µM forskolin instead of Gβγ was only inhibited by up to 20% (data not shown). Similarly, the peptide at 100 µM antagonized the stimulatory effects of Gβγ on the activity of the β-adrenergic receptor kinase and of phospholipase Cβ2 (figure 4b and c). These data indicate that multiple Gβγ-functions can be antagonized by the phosducin peptide, even though higher concentrations were required suggesting that the inhibitor molecule can be further optimized for different receptors.

### AC Assays

Adenylyl cyclase (AC) type 2 expressing membrane preparations (4-6 mg of membrane protein from Sf9 cells/assay tube) were incubated with GTPγS-activated rGₛα (100 nM) for 10 min at 30 C in 20 mM NaHepes (pH 8.0), 2 mM MgCl₂, and 1 mM EDTA. The different peptides were incubated with purified bovine brain Gβγ(100 nM) for 20 min at 4 C. Adenylyl cyclase activity was measured as described earlier (Pippig et al. 1993). The resulting [³²P]cAMP was purified by precipitation of ATP and chromatography on alumina.

### Phosphorylation of rhodopsin

Urea-treated rod outer segments containing >95% rhodopsin were prepared from bovine brain retina (Wilden and Kuehn 1982). 40 pmol rhodopsin were phosphorylated in a volume of 40 µl using 2.5 nM purified recombinant pARK-1 and 20 nM G-protein βγ-subunits from bovine brain as described earlier (Schroeder and Lohse 1996). Phosducin was present at 100 µM. The incubation was carried out at 30 C for 6 min under bright white light. The reaction was stopped, and the samples were resolved by SDS polyacrylamide gel electrophoresis. The absolute determination a of ³²P content of rhodopsin was done by excising the bands from the gel and Cerenkov counting.

### PLC Assays

Phospholipase Cβ2 activity was determined with a truncated version of PLC-β2 as described by Dietrich et al. (1994). [³H]Phosphatidyl 4,5-bisphosphate served as the substrate. When present the concentration of Gβγ was 600 nM, and that of Phd(215-232)100 µM.

### Example 3

### Structural basis of Gβγ-inhibition

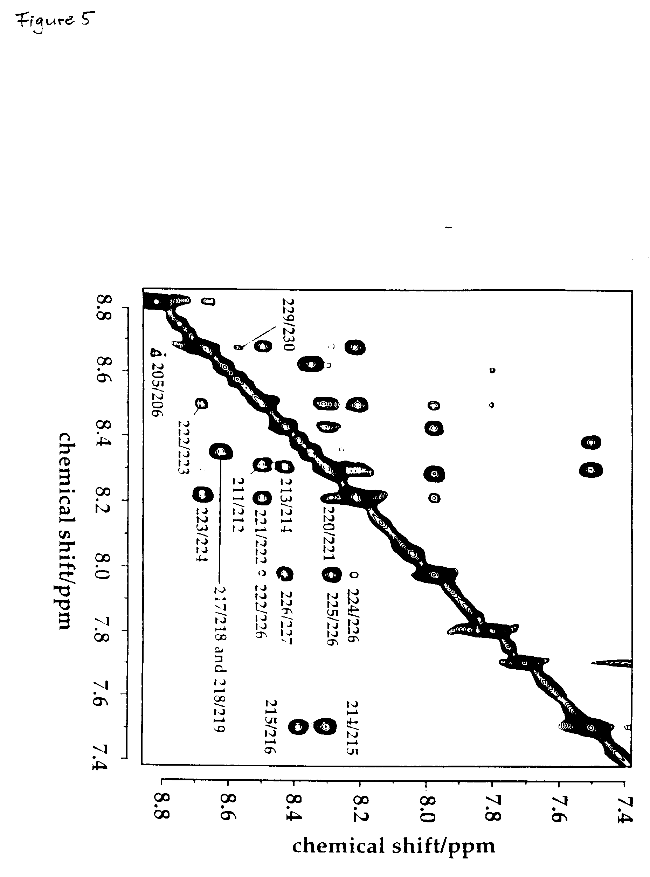

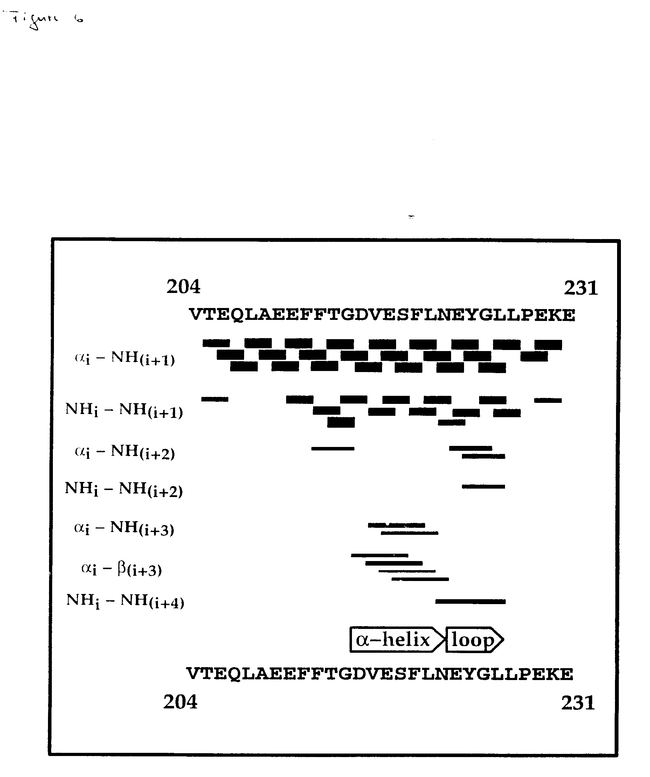

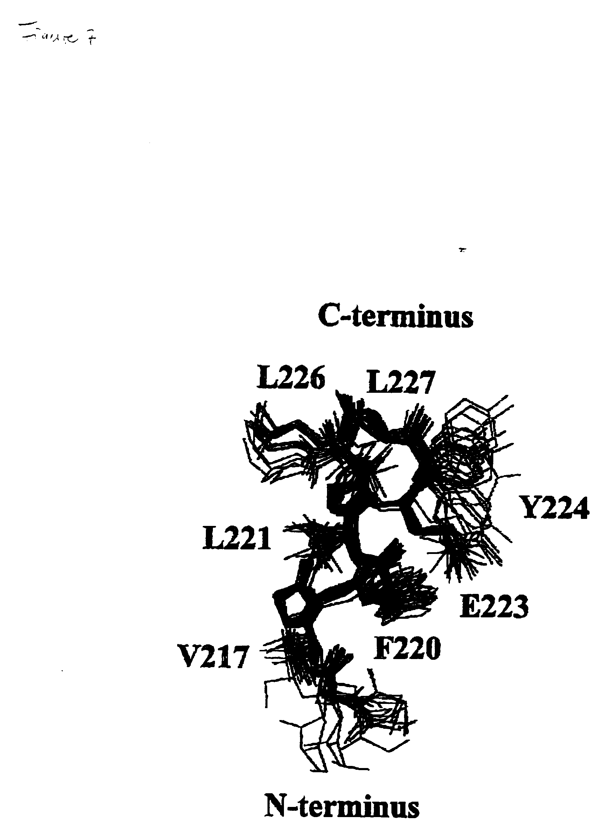

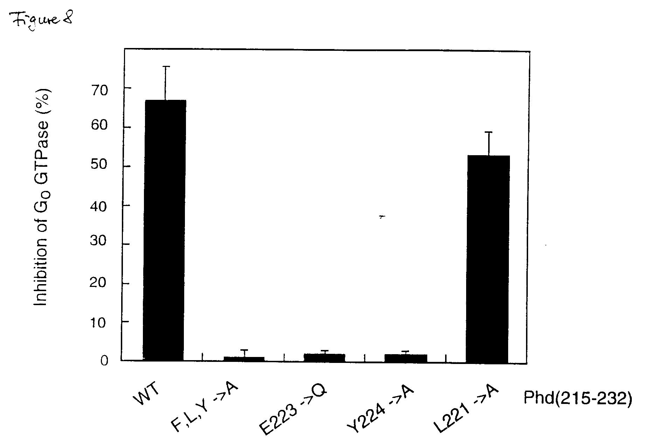

To elucidate the structural basis for the interaction of this region in phosducin with Gβγ, its structure was analyzed in NMR experiments with the peptide phosducin (204-231). These studies were done in a physiological buffer at pH 6.5. The two dimensional ¹H-NMR spectra showed a dominantly ordered structure in the region 216-226 as revealed by non-random coil chemical stuff values of a- and amide protons by NOEs of the type Hαi-Hβ(i+3) including residues 216-222, by long-range NOEs between F220. L221, N222 and L226 and by a large number of strong NH-NH NOEs (Figures 5/6). This ordered state is not populated to 100%, as can be seen from the simultaneous appearance of NH-NH and NH-Hα NOEs (Figure 6). This was taken into account using more generous upper bounds for this type of NOEs. Only sequential NOEs between amino acid protons (i) and the amide proton (i+1) and all medium and long range NOEs were used in structure calculations.
The NMR structure of the segment phosducin (215-226) (Figure 7) shows a short a-helical structure comprising residues 216-222 followed by a well ordered loop region formed by the amino acids up to 226. Hydrophobic interactions between the residues flanking the loop (F220 and L221 on the N-terminal and L226 on the C-terminal side) stabilize its structure. The side chains of E223 and Y224 are prominently exposed by the loop and presumably available for interactions.
The importance of this structured region in phosducin for the interaction with and inhibition of Gβγ was established in GTPase assays with a series of variations of the phosducin(215232) peptide carrying isolated or multiple amino acid exchanges (Figure 8). Replacement of the five hydrophobic residues located between positions 220 and 227 by alanine resulted in a peptide that was inactive. Replacement of the exposed tyrosine-residue (224) in the turn alone also caused a complete loss of activity, indicating the importance of the side chains of this loop. Likewise, abolishing the charge in the neighboring exposed position 223 by a change from glutamate to glutamine caused inactivity of the peptide. In contrast, replacement of L221 by A caused only a modes reduction of activity. These results support the notion that the exposed side chains of E223 and Y224 are essential for the interaction with Gβγ.

### NMR experiments and structure calculations

NMR samples contained 2 mM phosducin(204-231), 10 mM Na-phosphate, and 100 mM NaCl in 10% D2O/90%H2O or in pure D2O at pH 6.5. The spectra were recorded either on an AMX-500 or an AMX-600 spectrometer (Bruker). Al12D spectra were acquired in phase sensitive mode (TPPI, Marion and Wuethrich 1986) with appropriate suppression of the water resonance (WATERGATE pulse sequence, Piorto alt 1992). Clean TOCSY spectra (Griesinger et al. 1988) were measured using a TOWNY sequence for spin lock (Kadkhodaei et al. 1993). Mixing times used were 60 ms for the TOCSY and 40-250 ms for the NOESY experiments. 2D spectra were recorded at 277 K, with 2048 data points in the acquisition domain and 768-1024 data points in the t1 domain. Prior to Fourier transformation, the data were zero-filled to 2048 points in the t1 dimension and multiplied with appropriate window functions. Baseline corrections were performed in both dimensions using a Polynomial. Structures were generated using 87 sequential, 58 medium range, 13 long range NOEs and 4 hydrogen bond mimic distances with a standard simulated annealing protocol (Nilges et al. 1993) and an extended version of X-PLOR 3.851 (Bruenger et al 1993). Eighty structures were calculated starting from conformations with random backbone torsion angles. 9 NOEs per residue were applied in the amino acid range 216226. The averages of the X-PLOR potential energies found for the 20 structures with the lowest energies were 〈Eₜₒₜₐₗ〉 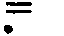 74 ± 9 kcal/mol, 〈E_{bonds}〉 = 4 ± 1 kcal/mol, 〈E_{angles}〉 = 44 ± 14 kcal/mol, 〈Eᵢₘₚᵣₒₚₑᵣₛ〉 = 7 ± 2 kcal/mol, 〈E_{vdW}〉 = 11 ± 31) kcal/mol, and 〈E_{NOE}〉 = 9 ± 2 kcal/mol. These structures fit with an average 〈R-m.s.d.〉 = 0.69.

### Example 4

### Molecular modeling of Gβγ- inhibitors

Taking advantage of the knowledge gathered by the description of the invention, it is possible to design new inhibitors by molecular modeling. The design may include e.g. conservative amino acid exchanges, introducing of peptido-mimetics or design of nonpeptidic chemical entities.
The structure of the Gβγ-phosducin complex is known by a resolution of 2.4 A, see Gaudet et al. 1996 which is hereby incorporated by reference. From the disclosure of this invention it is known that the important region of interaction with Gβγ correspond to the part of the Gβγ-phosducin complex where phosducin(217-231) interact with Gβγ. The preferred sequence part is FLNEYGLL and it is shown that E223 and Y224 of phosducin are on key positions. Some of the amino acids can be exchanged without dramatic loss of activity, e.g. L221->A. Knowing the important pattern for van der Waals interactions/ionic interactions and Hydrogen bonds essential for interaction together with steric restrictions and necessary hydrogen bonding (both from X-ray and NMR), it is possible to design e.g. by molecular modeling chemical entities fitting at the key regions of Gβγ. The critical contacts between phosducin and β-transducin are phosducin E223 / β-transducin T47, phosducin E223 / β-transducin R46, phosducin Y224 / β-transducin G310, phosducin Y224 / β-transducin R46. In addition, such chemical entities can than be optimized by comparison of their effect in the Gβγ assays. The structural refinement can also be supported by co-crystallization of Gβγ with such new inhibitors.

### Example 5

### Pharmaceutical compositions comprising Gβγ- inhibitors

The liquid or also the lyophilized pharmaceutical preparations may, if desired, contain conventional pharmaceutical auxiliary substances such as stabilizing agents or organic hydrophilic polymers.
Oligosaccharides such as sucrose, tetralose, lactose, dextrans with a molecular weight of about 10,000 to 2,000,000 are for example suitable as stabilizers. Organic hydrophilic polymers are macromolecules with a carbon backbone which is made up of hydrophilic monomeric units, if desired, with polar side groups such as polyethylene glycol or polyvinylpyrrolidone. The phammaceutical preparations additionally contain conventional pharmaceutical buffers such as alkali phosphates (sodium or potassium phosphate or their hydrogen or dihydrogen salts), salts of organic or inorganic acids or amino acids. The composition of the various buffer substances in the formulation is chosen so that a buffer capacity which is as low as possible results, of the injection or infusion solution ready for administration. This can be achieved by using an amount of buffer substances which is as low as possible and in doing so the total amount of the buffer should in particular not exceed a concentration of 100 mmol/l in the pharmaceutical solution. Buffer substances are preferably used at a concentration of 10 to 100 mmol/l and especially of 20 to 60 mmol/l. Alternatively, it is also possible to select the individual buffer substances such that they mutually compensate their action that is mainly in the acid or basic buffer range. In this case the total amount of buffer substances can be up to 200 mmol/l in the final administerable pharmaceutical preparation. The lyophilized pharmaceutical preparations preferably additionally contain a structure former which forms a crystalline matrix when the aqueous solution freezes, that also remains structurally stable during the subsequent lyophilization and during storage of the lyophilisate for longer periods under various external conditions. In this sense mannitol and glycine come into consideration as suitable structure formers.
The pharmaceutical preparations produced in this manner are preferably marketed in the form of lyophilisates. They can be used as single dose preparations, in which case a particular amount of the human protein is present in an injection bottle, ampoule or capsule and the lyophilisate is dissolved by the addition of an appropriate amount of reconstitution solution. The reconstitution solution can already contain the required amount of lye that is needed to adjust the desired pH value of the injectable solution. In addition conventional isotonic additives can also be used. The lyophilisate can, on the other hand, also contain wholly or partly the amounts of basic reagents required to set the advantageous pH range so that the reconstitution is carried out essentially with distilled water for injection purposes. Furthermore, the lyophilisate as well as also the reconstitution solution can contain agents which ensure the production of an isotonic solution. The reconstituted solution is then drawn up into an injection syringe and can be administered directly into the patient. The compounds of the invention are administered in amounts sufficient to inhibit Gβγ. This will usually be from about 0.1 mg to about 0.4g. per kilogram of body weight per day.
The compounds of the invention may be administered also as pharmaceutical acceptable salts, amides and esters and other compounds (prodrugs) which metabolize/hydrolyze in vivo to the pharmaceutically active compounds of the invention. Examples are inter alia Ethylesters, Sodium salts, Ammonium salts etc..

### References

Bauer PH, Mueller S, Puzicha M Pippig S. Obermaier B, Helmreich EJM, Lohse MJ (1992) Phosducin is a protein-kinase A-regulated G-protein regulators *Nature*, 358, 73-76.
Bemman DM, Wilkie T Vl Gilman AG (1996) GAIP and F∼S4 are GTPase activating proteins (GAPs) for the Gi subfamily of G protein a subunits. Cell, 86, 445452.
Bimbaumer L (1992) Receptor-to-effector signaling through G proteins: roles for βγ dimers as well as a subunits. Cell, 71. 1069-1072.
Bruenger AT (1993) X-PLOR Manual. New Haven: Yale University Press.
Chen J, DeVivo M, Dingus J. Harry A. Li J, Sui J, Carty DJ, Blank Jl, Exton JH, Stoffel RH, Inglese J, Lefkowitz RJ, Logothetis DE, Hildebrandt JD, Iyengar R (1995) A region of adenylyl cyclase 2 critical for regulation by G protein βγ subunits. Science, 268, 1166-1169. Danner S, Lohse MJ (1996) Phosducin is a ubiquitous G-protein regulators *P'oc.Natl. Acad Sci.* USA, 93, 10145-10150.
Dietrich A, Meister, Brazil D. Camps M. Gierschik P (1994) Stimulation of phospholipase Cβ2 by recombinant guanine-nucleotide-binding protein βγ dimers produced in a baculovirus/insect cell expression system. Requirement of γ-subunit isoprenylation for simulation of phospholipase C. *Eur. J Biochem*, 219, 171-178.
Gaudet R, Bohm A, Silger PB (1996) Crystal structure at 2.4 A resolution of the complex of transducin βγ and its regulator, phosducin. Cell, 87, 577-588.
Griesinger C, Otting G, Wothrich K. Ernst RR (1988) Clean TOCSY for 1H spin system identification in macromolecules- J *Am Chem Soc., 110*. 7870-7872.
HausdorffWP. Caron MG. Lefkowitz RJ (1990) Turning off the signal: desensitization of p-adrenergic receptor function. *FASEB* J. 4. 2881-2889.
Hawes BE, Touhara K. Kurose H. Lefkowitz RJ, Inglese J (1994) Determination of the Gβγ binding domain of phosducin. A regulatable modulator of Gβγ signalling. J Biol Chem, 269, 29825-29830.
Hepler JR, Gilman AG (1992) G proteins. Trends Biochem Sci, 17, 383-387.
Higashijima T, Burniex J. Ross EM (1990) Regulation of Gi and Goby mastoparar, related amphiphilic peptides, and hydrophobic amines. Mechanism and structural determinants of activity. J. Biol, Chem. 265, 14176-14186.
Hunt TW, Fields TA Casey PJ, Paalta EG (1996) RGS 10 is a selective activator of Gai GTPase activity. Nantre, 383, 175-177.
Iniguez-Lluhi J, Kleuss C, Gilman AG (1993) The ir.aportance of G protein βγ subunits. Trends Cell Biol. 3, 230-236.
Kadkhodaei M, Hwang T-L Tang J, Shaka AJ (1993) A simple windowless mixing sequence to suppress cross-relaxarion in TOCSY experiments. J. Magnet. Res., 105, 104-107.
Koch WJ, Inglese J, Stone WC, Lelkowits Rl (1993) The binding site for the βγ subunits of heterotrimeric G proteins on the 3-adrenergic receptor kinase-J Biol Chem., 268, 82568260.
Lee RH, Lieberman BS, Lolley RN (1987) A novel complex from bovine visual cells of a 33000-dalton phosphoprorein with p- and y-transducin: purification and subunit structure Biochemistry, 26.3983-3990.
Lee RH, Ting TD, Lieberman BS, Tobias DE Lolley RN, Ho Y-K (1992) Regulation of retinal cGMP cascade by phosducin in bovine rod photoreceptor cells. Interaction of phosducin and transducin. J. BIQI. Chem., 267, 25104-25112.
Lohse MJ (1993) Molecular mechanisms of membrane receptor desensitization. Biochim. Biophys. Acta, 1179. 171-188.
Lohse MJ, Andexinger S, Pitcher J, Trukawinsid S, Codina J, Faure J-P, Caron MG, Lefkowitz RJ (1992) Receptor-specific desensitization with purified proteins: kinase dependence and receptor-specificity of β-arrestin and arrestin in the β2-adrenergic receptor and rhodopsin systems. J. Biol. Chem., 267, 8558-8564.
Lohse MJ, Kok K-N, Schwabe U (1986) Agonist photoaffinity labeling of Al adenosine receptors: Persistent activation reveals spare receptors. Mol. Pharmacol., 30, 403-409.
Marion D and Wuethrich K (1986) Application of phase-sensitive two dimensional correlated spectroscopy (C(SY)) for measurements of 1 H-1 H spin-spin coupling constants in proteins. Biphys. Biochem. Res. Commun., 113, 967-974
Mueller S. Lohse MJ (1995) The role of G-protein βγ subunits in signal transduction. Biochem. Soc. Transactions, 23. 141-148.
Mueller S. Hekman M, Lohse MJ (1993) Specific enhancement of p-adrenergic receptor kinase activity by definal G-protein β and γ subunits. Proc. Natl. Acad. Sci. USA, 90, 10439-10443.
Mueller S. Straub A. Schroeder S, Bauer P, Lohse MJ (1996) Interactions of phosducin with defined G protein βγ-subunits. J. Biol. Chem., 271, 11781-11786. ∼ Neer FJ (1995) Hetetotrimeric G proteins: Organizers of transmembrane signals. Cell, 80, 249-257.
Nilges M (1993) A calculation strategy for the structure determination of symmetric dimers by 1H NMR Proteins, 17, 297-309.
Offermanns S, Schultz G (1994) Naunyn-Schmiedeberg's Arch. Pharmacol., 350, 329-338. Piotto M. Saudek V. Sklenar V (1992) Gradient-tailored excitation of single-quantum NMR spectra of aqueous solutions. J. Biomol. NMR, 2, 661-664.
Pippig S, Andexinger S, Daniel K, Puzicha M, Caron MG, Lefkowitz RJ, Lohse MJ (1993) Overexpression of β-adrenergic receptor kinase and p-arrestin augment homologous desensitization of β2-adrenergic receptors, J. Biol. Chem, 268, 3201-3208.
Pitcher JA, Inglese J, Higgins JB, Aniza JL, Casey PJ, Kim C, Benovic JL, Kwatra MM, Caron MG, Lefkowitz Rl (1992) Role of βγ-subunits of G proteins in targeting the padrenergic receptor kinase to membrane-bound receptors. Science, ZS7, 1264-1267.
Reig JA, Yu L, Klein DC (1990) Pineal transduction adrenergic ->cyclic AMP-dependent phosphorylation of cytoplasmic 33-kDa protein (MEKA) which binds βγ-complex of transducin. J. Biol. Chem. 265. 5816-5824.
Schroeder S, Lohse MJ (1996) Inhibition of G-protein pv-subunit functions by phosducin-like protein. Proc. Natl. Acad. Sci. USA 93. 2100-2104.
Schulz K, Danner S Bauer P, Schroeder S, Lohse MJ (1996) Expression of phosducin in a phosducin-negative cell fine reveals functions of a Gβγ-binding protein. J. Biol. Chem., 271, 21546-22551.
Sternweis PC, Robishaw ID (1984) Isolation of two proteins with high affinity for guanine nucleotides from membranes of bovine brain. J. Biol. Chem., 259, 13806-13813.
Strittmatter SM, Valenzuela D, Sudo Y. Linder ME, Fishman MC (1991) An intracellular guanine release protein for Go GAP43 stipulates isolated a subunits by a novel mechanism. J. Biol. Chem. 266. 22465-22471.
Touhara K. Inglese J, Pitcher JA. Shaw G, Lefkowitz RJ (1994) Binding of G protein βγ subunits to pleckstrin homology domains. J. Biol. Chem, 269, 10217-10220.
Watson N, Linder ME. Druey KM. Kehrl JH, Blumer KJ (1996) RGS famity members: GTPase activating proteins for heterotrimeric G-protein a-subunits. Nature, 383, 172-175. Weng G, Li J. Dingus J. Hildebrand ID, Weinstein H, Iyengar R (1996) Gp subunits interacts with a Peptide encoding region 956-982 of adenylyl cyclase 2. J. Biol. Chem., 271, 2644526448.
Wilden U, Kuehn H (1982) Light-dependent phosphorylation of rhodopsin. Number of phosphorylation sites. Biochemistry, 21, 3014-3022.
Xu J. Wu D. Slepak VZ, Simon AG (1995) The N-terminus of phosducin is involved in binding of βγ subunits of G protein. Proc. Natl. Acad. Sci. USA, 92, 2086-2090.
Yoshida T, Willardson BK Wilkins JE, Jensen GJ, Thornton, BD, Bitensky MW (1994) The phosphorylation stete of phosducin determines its ability to block subunit interactions and inhibit transducin binding to activated rhodopsin. J. Biol. Chem, 269, 24050-24057.

## Claims

1. A compound which interacts with Gβγ as an inhibitor, wherein said compound has a molecular weight equal to or less than the molecular weight of phosducin(217-246).

2. A compound of claim 1, which interacts as an allosteric inhibitor.

3. A compound of one of the claims 1 or 2, wherein said compound has a molecular weight equal to or less than the molecular weight of phosducin(213-233).

4. A compound of one of the claims 1 to 3, wherein the compound interacts with Gβγ as an inhibitor in a manner equivalent to the interaction of E223 and Y224 of phosducin with Gβγ.

5. A compound of one of the claims 1 to 4 comprising the peptide sequence EY.

6. A compound of one of the claims 1 to 5 comprising the peptide sequence FLNEYGLL.

7. A pharmaceutical composition comprising at least a compound of one of the claims 1 to 6, its pharmaceutical acceptable salts, amides and esters in addition to common carrier substances and auxiliary substances.

8. Use of a compound as claimed in one of the claims 1 to 6 for the production of pharmaceutical agents having inhibitory action on Gβγ.

9. Use of compound as claimed in one of the claims 1 to 6 for the production of pharmaceutical agents for treating cardiovascular disorders.

10. Use of compound as claimed in one of the claims 1 to 6 for the production of pharmaceutical agents for treating tumors or disorders of the nervous system.
